# EUROPEAN PATENT SPECIFICATION

(11) **EP 2 031 390 B1**
(45) Date of publication and mention of the grant of the patent: **16.07.2014**
(21) Application number: 08010094.4
(22) Date of filing: 18.08.2004
(51) Int. Cl.: G01N 33/50, C12N 9/96, C07K 14/415, C07K 14/425

(54) **Stabilizing agent and blocking agent**
Stabilisator und Blocker
Agent de stabilisation et agent de blocage

(30) Priority: 20.08.2003 JP 2003296005; 26.03.2004 JP 2004093863; 02.07.2004 JP 2004197049
(43) Date of publication of application: 04.03.2009
(62) Divisional of application: 04019560.4
(73) Proprietor: SEIKAGAKU CORPORATION, Chuo-ku, Tokyo 103-0023 (JP)
(72) Inventor: Ishimaru, Tsuyoshi, Higashiyamato-shi Tokyo 207-0003 (JP); Miyaura, Shuichi, Yokohama-shi Kanagawa 225-0002 (JP)
(74) Representative: Schwanhäußer, Gernot

(56) References cited:
- EP-A- 0 745 670
- US-A- 3 575 864
- US-A- 4 960 692

## Description

### BACKGROUND OF THE INVENTION

### Field of the Invention

The present invention relates to the use of a stabilizing agent for storing a substance capable of forming a specific binding pair (hereinafter sometimes referred to as a specific-binding-pair-forming substance), the stabilizing agent containing as an active ingredient a polypeptide derived from a plant (hereinafter sometimes referred to as a plant-derived polypeptide).

### Description of the Related Art

Functions of proteins such as antibodies and antigens, which are specific-binding-pair-forming substances present in organisms, are known to be related to a stereostructure thereof, and the functions are known to be inactivated as a result of change in stereostructure. Particularly in an antigen-antibody reaction, utmost care must be taken for maintaining a stereostructure of an antibody during its storage, since the stereostructure of a variable region or a complement binding region (Fc region) of an antibody which specifically binds to an antigen plays an important role. Therefore, at present, antibodies are usually stored in a frozen state, after they are mixed with polyethylene glycol or bovine serum albumin (hereinafter sometimes referred to as BSA).

Meanwhile, antibodies and antigens are assayed generally through a method such as immunoassay.

When an immunoassay is performed, an antibody or an antigen is immobilized on a solid support such as a plate, beads, or latex particles. A portion of the solid phase where an antibody or an antigen has not been bound is coated with a blocking agent in order to prevent nvn-specific adsorption of the antibody or the antigen serving as an assay target. The aforementioned BSA is widely employed as the blocking agent

When a specimen such as nucleic acid or protein is caused to be bound to a specific-binding substance immobilized on a support in a system for detecting the specimen, a blocking agent predominantly containing casein is employed in order to prevent non-specific adsorption of the specimen to the support (Japanese Patent Application Laid-Open (kokai) No. 6-160385).

EP 0745670 A1 corresponding to WO 96/11264 discloses a technique for stabilizing a protein without employing BSA. Specifically, In the method, a hydrolyzate of protein derived from wheat, soybean, etc. is employed as a stabilizing agent for transglutaminase, which is an enzyme widely employed in the food processing industry. However, in some cases, functions of antibodies are lost more easily as compared with those of enzymes, and therefore, a technique applicable to an enzyme is not always employed with respect to an antibody. Furthermore, protein is generally considered to have lower stability in solution than in a dry state. Thus, stabilization effect which is attained in a dry state is not considered to be adapted to the solution state. In this connection, WO 96/11264 refers only to the case of stabilization in a dry state. Accordingly, the document neither discloses nor suggests a stabilizing agent for an antibody or possibility thereof, and moreover, neither discloses nor suggests a stabilizing agent for maintaining stability in solution.

BSA, which is generally employed at present as a stabilizing agent for a specific-binding-pair-forming substance or a blocking agent for preventing non-specific adsorption during an assay employing a specific-binding-pair-forming substance, is a protein derived from bovine blood. In general, although not employed as a drug, a protein derived from body fluid or a secretion of animals must be handled with care in consideration of zoonosis (in the bovine, bovine spongiform encephalopathy (BSE), foot-mouth disease, or other diseases). Since the function of an antibody is readily lost, even when BSA bas been added, antigen-binding ability of the antibody considerably decreases within a few days when the antibody is maintained at room temperature in the form of solution. Therefore, there is keen demand for development of a stabilizing agent which exhibits an excellent stabilizing effect and prevention performance with respect to non-specific adsorption of a substance employed in an assay employing a specific-binding-pair-forming substance such as an antibody or an antigen, and which does not raise any problem of potential infection with a pathogen of BSE or other diseases.

### SUMMARY OF THE INVENTION

The present inventors have carried out extensive studies in order to solve the aforementioned problems, and have found that a plant-derived polypeptide can remarkably enhance stability of a specific-binding-pair-forming substance derived from an organism, and that the polypeptide can be employed as a stabilizing agent during an assay employing a specific-binding-pair-forming substance. The present invention has been accomplished on the basis of these findings.

Accordingly, in the present invention, there is provided the use of a stabilizing agent for a specific-binding-pair-forming substance, the agent comprising a plant-derived polypeptide as an active ingredient.

The specific-binding-pair-forming substance may be a substance employed in an assay for a biological material.

The specific-binding-pair-forming substance is an antibody or an antigen.

The plant-derived polypeptide is obtained by degradation of a plant-derived protein.

The plant is an agricultural crop.

The plant-derived polypeptide is obtained from, at least one plant selected from the group consisting of soybean, wheat, corn, potato, and rice.

The protein may be at least one species selected from the group consisting of gliadin, zein, glutenin, gluten, hordein, oryzenin, glycinin, patatin, and conglycinin.

In the invention there is provided the use of a stabilizing composition comprising the aforementioned stabilizing agent, and an alkali or a buffer.

The stabilizing composition may be in the form of sterilized solution through filtration, with a pH of 3.0 to 9.0.

The stabilizing composition is preferably used for immunoassay.

There is also described a blocking agent for preventing non-specific adsorption in an assay employing a specific-binding-pair-forming substance, the blocking agent comprising as an active ingredient a plant-derived polypeptide.

Preferably, the plant-derived polypeptide is obtained by degradation of a plant-derived protein.

Preferably, the plant is an agricultural crop.

Preferably, the plant-derived polypeptide is obtained from, at least one plant selected from the group consisting of soybean, wheat, corn, potato, and rice.

The protein may be at least one species selected from the group consisting of gliadin, zein, glutenin, gluten, hordein, oryzenin, glycinin, patatin, and conglycinin.

The blocking agent may exhibit an effect of stabilizing a specific-binding-pair-forming substance.

The specific-binding-pair-forming substance may be a substance employed in an assay for a biological material.

The specific-binding-pair-forming substance may be an antibody, an antigen, or nucleic acrid.

In the present invention, there is provided the use of a composition in an assay employing a specific-bhuling-pair-forming substance, the composition comprising the specific-binding-pair-forming substance, and the aforementioned stabilizing composition.

The specific-binding-pair-forming substance maybe a substance employed in an assay for a biological material.

The specific-binding-pair-forming substance is an antibody or an antigen.

There is also described a composition for use in an assay employing a specific-binding-pair-forming substance, the composition comprising the specific-binding-pair-forming substance and the aforementioned blocking agent

The specific-binding-pair-forming substance may be a substance employed in an assay for a biological material.

The specific-binding-pair-forming substance may be an antibody, an antigen, or nucleic acid.

The antibody or the antigen may be a complex with a labeling substance for use in immunoassay.

The aforementioned compositions is in the solution form.

In the present invention, the use includes a method for stabilizing a specific-binding-pair-forming substance, the method comprising bringing a stabilizing agent or a stabilizing composition to coexist with the specific-binding-pair-forming substance.

There is also described a method for blocking non-specific binding of a specific-binding-pair-forming substance to a solid phase, the method comprising coating the solid phase which is immobilized with a specific-binding-pair-forming substance with the aforementioned blocking agent, thereby preventing non-specific binding, to the solid phase, of a substance which forms a complex with the specific-binding substance in a sample.

There is also described a kit for use in an assay employing a specific-binding-pair-forming substance, the kit comprising any of the aforementioned compositions.

There is also described a kit for use in an assay employing a specific-binding-pair-forming substance, the kit comprising a solid phase onto which any of the aforementioned blocking agents has been coated.

In the present invention, there is provided use of a plant-derived polypeptide as a stabilizing agent for a specific-binding-pair-forming substance serving as an assay reagent.

### DETAILED DESCRIPTION OF PREFERRED EMBODIMENTS

Best modes for carrying out the present invention will next be described.

### 1. Stabilizing agent used in the present invention

The stabilizing agent used in the present invention for a specific-binding-pair-forming substance for use in an assay for a biological substance or a similar substance contains a plant-derived polypeptide as an active ingredient.

The stabilizing agent used in the present invention stabilizes a specific-binding-pair-forming substance. As used herein, the term "specific-binding-pair-forming substance" refers to one member of a pair of substances which form a binding pair through specific interaction (affinity) provided between biological substances. Specifically, the term refers to one substance of a pair, namely an antibody and an antigen. No particular limitation is imposed on the origin and type of the specific-binding-pair-forming substance, and the substance is preferably employed for assaying a biological substance. The antigen is preferably an antigen for use in immunoassay; e.g., protein.

In the present invention, the expression "stabilization of a specific-binding-pair-forming substance" refers to stabilization of a specific-binding-pair-forming substance during storage in an aqueous solution containing an alkali or buffer. More specifically, the expression refers to a function for preventing breakage of a specific binding site and decrease in titer of specific-binding-pair-forming substances such as antibodies and antigens during long-term storage.

The term "plant" in relation to the stabilizing agent of the present invention refers to an agricultural product. The plants include wheat, corn, Japonica rice, Indica rice, Javanica rice, African rice, and glutinous rice, and soybean, potato. Of these, soybean and corn are most preferred.

The "plant-derived polypeptide" contained in the stabilizing agent of the present invention is a polypeptide which is produced through degradation of the aforementioned plant-derived protein. The aforementioned plant-derived protein includes a protein extracted from the above plant or a fraction containing the protein. Specific examples include reserve proteins such as gliadin, zein, glutenin, gluten, hordein, oryzenin, glycinin, patatin, and conglycinin; functional proteins such as lectin, amylase, and enzymes involved in respiration and photosynthesis; and structural proteins derived from roots, stalks, leaves, flowers, fruits, seeds, etc. Of these, reserve proteins are particularly preferred. In order to obtain the polypeptide from the protein, the protein is degraded. No particular limitation is imposed on the method of degradation, so long as the method attains the object for obtaining polypeptide through reduction of molecular weight, and examples thereof include enzymatic hydrolysis, acid-hydrolysis, and alkali-hydrolysis. Any enzyme can be employed for hydrolysis, so long as the enzyme belongs to the protease group and is able to degrade protein to form polypeptide. Examples of the enzyme include animal-derived protease such as pepsin, trypsin, and chymotrypsin; plant-derived protease such as papain, ficin, and bromelain; and other proteases, derived from microorganisms such as bacteria, molds, and actinomycetes. Examples of the acid employed in acid-hydrolysis include hydrochloric acid, sulfuric acid, phosphoric acid, and nitric acid, and examples of the alkali substance employed in alkali- hydrolysis include sodium hydroxide, potassium hydroxide, and sodium carbonate. The polypeptide formed through hydrolysis preferably has a weight average molecular weight of 200 Da to 300,000 Da, more preferably 300 Da to 200,000 Da. When protein is subjected to hydrolysis, amino acid is formed. The stabilizing agent used in the present invention may contain amino acid, so long as the agent contains polypeptide as an essential component. Furthermore, the stabilizing agent used in the present invention may contain a plurality of polypeptides in combination, the polypeptides being obtained from one or more proteins derived from one or more different plant species or from a single plant species. The stabilizing agent of the present invention and another stabilizer (e.g., sugar or surfactant) may be used in combination.

The plant-derived polypeptides include a soybean protein hydrolyzate, a wheat protein hydrolyzate and derivatives thereof, a potato protein hydrolyzate, a corn protein hydrolyzate, and a rice bran protein hydrolyzate. Of these, a corn protein hydrolyzate is particularly preferred. Among corn protein hydrolyzates, a peptide having a molecular weight of 200 Da to 4,000 Da and a free amino acid content with respect to the total amino acid content of 1% or less is preferred. Moreover, a peptide having the following amino acid composition (wt.%) is more preferred; aspartic acid 2.5 to 12.5, threonine 2.5 to 6.0, serine 4.0 to 6.0, glutamic acid 15.0 to 50.0, glycine 2.0 to 5.5, alanine 2.0 to 13.5, valine 4.0 to 8.0, cysteine 0.0 to 1.5, methionine 1.0 to 2.0, isoleucine 3.0 to 6.0, leucine 6.0 to 15.0, tyrosine 1.0 to 4.0, phenylalanine 2.0 to 5.5, lysine 0.5 to 7.0, histidine 0.5 to 3.0, arginine 1.0 to 8.0, and proline 5.0 to 13.0.

The hydrolyzates and some typical characteristics are as follows:
<1> Soybean protein hydrolyzate
   Appearance: pale yellow to brown
   Total nitrogen content (%): 2.6 to 3.4
   pH: 3.8 to 6.2
<2> Soybean protein peptone
<3> Soybean protein enzymatically hydrolyzed product
<4> Soybean protein acid-hydrolyzed product
<5> Wheat protein hydrolyzate
   Appearance: brown liquid
   Weight loss by drying (%): 75 to 80
   Total nitrogen amount (%): 2.7 to 3.5
<6> Wheat protein hydrolyzate derivative
   Appearance: Grayish white
   Total solid content (%); 81.3 to 1.00
   Nitrogen (%): 13.0 to 16.0
   Ashing residue (%): 13.5 or less
   pH: 3.5 to 4.5
   Water content (%): 5.0 or less
<7> Potato protein hydrolyzate
   Appearance: yellow
   Total solid content (%): 24.0 to 28.0
   Nitrogen content (%): 2.5 to 4.0
   Ashing residue (%): 4.5 or less
   pH :4.0 to 5.0
   Molecular weight: 600
<8> Corn protein hydrolyzate
   Appearance: White or pale yellow
   Water content (%): 5.0 or less
   Crude ash (%): 2.0 or less
   Crude protein (%): 90.0 or more
   Sugar content (%): 5.0 or less
   Heavy metals (ppm): 4.0 or less
   Arsenic (ppm): 1.0 or less
   Molecular weight distribution: about 2 to 10 (oligopeptide)
   Amino acid composition (wt.%):glutamic acid 24.67, leucine 13.69, alanine 12.99, proline 9.69, aspartic acid 6.04, serine 5.33, valine 4.94, threonine 3.95, isoleucine 3.77, tyrosine 3.42, glycine 2.39, phenylalanine 2.00, methionine 1.45, arginine 1.21, cysteine 1.07, lysine 1.00, and histidine 1.00.
<9> Rice bran protein hydrolyzate
   pH: 6.5 to 7.5
   Molecular weight: 150,000

The stabilizing agent used in the present invention is also capable of stabilizing a complex of an antibody or an antigen with a labeling substance for use in immunoassay. Examples of the above labeling substance include an enzyme, avidin, streptavidin, biotin, radioisotope, a fluorescent substance, a luminescence substance, and metal colloid.

The stabilizing agent composition used in the present invention contains the aforementioned stabilizing agent, and an alkali or a buffer in the solution form.

In other words, the stabilizing agent used in the present invention may be dissolved in an aqueous solvent containing an alkali, thereby providing a solution-form stabilizing agent. Examples of the alkali include alkali metal hydroxides and alkaline earth hydroxides. Of these, alkali metal hydroxides are particularly preferred. The alkali metal hydroxide is a hydroxide of an alkali metal such as potassium, lithium, or sodium and includes potassium hydroxide, lithium hydroxide, and sodium hydroxide. These alkali metal hydroxides may be used singly or in combination of two or more species, in the form of aqueous solution. Among the alkali metal hydroxides, sodium hydroxide is most preferred. The concentration of alkali metal hydroxide is generally 5 mmol/L to 2 mol/L, preferably 10 mmol/L to 500 mmol/L. The aforementioned aqueous solution generally has a pH of 3.0 to 9.0, preferably 6.5 to 8.0, more preferably 6.8 to 72.

In use, the stabilizing agent of the present invention may be dissolved in a buffer (buffer solution). Although no particular limitation is imposed on the type of the buffer, buffers such as a phosphate buffer, a Tris-HCl buffer, a Good's buffer, and a borate buffer are preferred. These buffers may be used singly or in combination of two or more species, and in an arbitrary amount.

Alternatively, the stabilizing agent of the present invention may be dissolved in an alkali metal hydroxide solution or a buffer so as to regulate the pH of the solution, and filtered for sterilization by means of a 0.22-µm filter or a similar filter, to thereby obtain a stabilizing agent composition employable in the present invention.

The stabilizing agent or the stabilizing agent composition used in the present invention generally has a polypeptide concentration of 0.01 to 40% (w/v), preferably 0.1 to 30% (w/v).

The stabilizing agent or the stabilizing agent composition of the present invention stabilizes a specific-binding-pair-forming substance; namely an antibody or an antigen, so as to maintain the titer thereof at a high level. In a specific procedure, upon storage of an antibody or an antigen, the stabilizing agent or the stabilizing agent composition of the present invention is added to the antibody or the antigen such that the polypeptide concentration is adjusted to 0.01 to 10% (w/v), to thereby form a composition in the form of solution. The antibody or the antigen can be reliably stored in the solution. In actual use, the stabilizing agent or the stabilizing agent composition of the present invention may be added to a solution (e.g., phosphate buffered saline (PBS) solution) containing, for example, an antibody in an amount of 1 ng/mL to 10 mg/mL.

In addition to the stabilizing agent used in the stabilizing composition of the present invention and a specific-binding-pair-forming substance, the aforementioned composition may further contain other stabilizing agents, a preservative, a surfactant, a sugar, a polyhydric alcohol (e.g., glycerol), a hydroxyl-group-containing polyether (e.g., polyethylene glycol (PEG)), etc.

Examples of the surfactant include non-ionic surfactants. Among them, polyoxyethylene sorbitan fatty acid esters (Tween series surfactants), polyoxyethylene p-t-octylphenyl ethers (Triton series surfactants), etc. are preferred. Examples of most typically employed species include Tween 20 and Triton X-100. When such a surfactant is incorporated into a reaction system where two substances react to form specific binding pairs (e.g., reaction system of antigen-antibody reaction, the blank level in analysis is lowered. Alternatively, when a surfactant is caused to be present during solid-liquid separation (e.g., washing) performed after completion of reaction between one substance of the specific-binding-pair substances immobilized on a solid phase and the other free substance, the blank level can also be lowered.

Examples of the sugar include monosaccharides, disaccharides (e.g., sucrose, maltose, lactose, and trehalose), sugar alcohols (e.g., sorbitol), oligosaccharides (e.g., raffinose and maltotriose), and water-soluble polysaccharides (e.g., cyclodextrin, water-soluble cellulose, pullulan, and carrageenan).

The thus-prepared composition may be incorporated, as a component, into a kit employing an antibody (e.g., a kit for immunoassay) or a kit employing a nucleic acid probe which does not form part of the invention. The composition is remarkably useful in that the quality of the kit can be maintained for a very prolonged period of time.

The stabilizing agent of the present invention may be employed as the below-mentioned blocking agent. Upon use, the stabilizing agent serves as a blocking agent for preventing non-specific adsorption, which agent stabilizes a specific-binding-pair-forming substance. Thus, the stabilizing agent used in the invention is remarkably useful.

### (2) Blocking agent

The blocking agent described herein contains a plant-derived polypeptide as an active ingredient and prevents non-specific adsorption in an assay employing a specific-binding-pair-forming substance. As used herein, the term "blocking agent" refers to a substance which prevents non-specific binding of a target substance to be determined by a specific-binding-pair-forming substance to a solid phase (or carrier) on which the specific-binding-pair-forming substance has been immobilized, the non-specific binding disturbing accurate measurement of the target substance.

In relation to the blocking agent described herein, the terms "plant," "plant-derived polypeptide," and "degradation" have the same meanings as defined in relation to the aforementioned stabilizing agent used in the present invention.

In use for an assay employing a specific-binding-pair-forming substance, the blocking agent is coated on a solid phase so as to prevent non-specific binding, to the solid phase, of a substance which forms a complex with a specific-binding substance in a sample, while the specific-binding-pair-forming substance (e.g., specifically binding protein, an antibody, an antigen, or nucleic acid, with an antibody being particularly preferred) has been immobilized on the solid phase (or carrier). The form of the solid phase may be a plate, beads, latex particles, or a membrane, with a plate being particularly preferred. Examples of the material for the solid phase include polystyrene, nitrocellulose, cellulose acetate, silica gel, glass, metal, semiconductor, silicon, a magnetic carrier, ceramics, paper, and cloth, with polystyrene being preferred. No particular limitation is imposed on the method of fixing the blocking agent on the solid phase, and any adsorption method, such as physical adsorption or chemical adsorption, maybe employed. Preferably, fixation is performed through generally employed physical adsorption.

The composition for use in an assay employing a specific-binding-pair-forming substance contains the blocking agent and the specific-binding-pair-forming substance. In one embodiment, the blocking agent is coated on a portion of the solid phase on which a specific-binding-pair-forming substance (an antibody, an antigen, or nucleic acid being particularly preferred) has been immobilized and no specific-binding-pair-forming substance has been bound, whereby non-specific binding of the specific-binding-pair-forming substance to the portion is prevented. The aforementioned composition may contain the blocking agent and a complex in which a labeling substance employed in immunoassay, a nucleic acid probe, or a nucleic acid chip is bound to the aforementioned antibody, antigen, or nucleic acid.

When the blocking agent is coated on a plate, the following procedure maybe employed. Specifically, a plate on which an antibody or a similar substance has been immobilized is washed several times with, for example, PBS. Subsequently, PBS containing the blocking agent (0.1 to 10% (w/v)) is portionwise poured onto each well of the plate, followed by incubation at 2 to 40°C for 10 minutes to 24 hours. After completion of incubation, the solution is removed, and the plate is washed several times with PBS (optionally containing a nonionic surfactant such as Tween 20) or a similar material. Through the above procedure, there can be produced a plate on which the blocking agent has been coated. The aforementioned plate on which an antibody or a similar substance has been immobilized is incubated with the blocking agent, followed by removal of the solution and naturally drying, to thereby produce a component The component may be incorporated into a kit for assaying, for example, an antigen which is specifically bound to an antibody. Such a plate is remarkably useful in that non-specific binding to the solid phase can be prevented and that the quality of the antibody can be maintained for a very prolonged period of time. The kit may further include the aforementioned stabilizing agent used in the present invention as an essential component.

### EXAMPLES

### Example 1

Studies on stabilizing effect of the stabilizing agent used in the present invention on an enzyme-labeled antibody

### 1. Methods

### <1> Preparation of an enzyme-labeled antibody solution and storage thereof

Each of the test substances (stabilizing agent) listed in Table 1 was added to a 50 mmol/L Tris-HCl buffer (Tris-HCl: pH 7.3 to 7.7) containing 0.15 mol/L sodium chloride, 0.05% Tween 20, and 0.05% ProClin 300 (preservative) (hereinafter the buffer is referred to as T-TBS) so that a desired test substance concentration was attained. The mixture was filtered by means of a 0.22-µm filter, to thereby obtain a test substance solution. Horseradish peroxidase (BRP)-labeled goat anti-mouse IgG antibody (product of Jackson, hereinafter referred to as HRP-anti-mouse IgG antibody) was diluted 10,000 fold with the aforementioned test substance solution, and the diluted solution was tested. Stability of HRP-anti-mouse IgG antibody was investigated on the day of preparation (day 0), 5 days after storage at 37°C (day 5), and 12 days after storage at 37°C (day 12). Each test solution was cooled to room temperature before measurement of antibody activity.

### <2> Measurement of activity of enzyme-labeled antibody

### (i) Preparation of a goat anti-mouse IgG antibody immobilized plate

A goat anti-mouse IgG antibody (product of Jackson) was diluted with phosphate buffered saline (pH 7.2 to 7.5, divalent-ion-free (e.g., Ca-ion-free); hereinafter referred to as PBS (-)) to 20 µg/mL. The thus-prepared solution was added in an amount of 50 µL to each well of a Nunc-immuno plate (Product name; Maxi soap, product of Nunc) and stored at 4°C for 14 to 18 hours, whereby the well was uniformly coated with the antibody. The plate was washed twice with PBS (-). In order to block a portion of the plate which had not been coated with the goat anti-mouse IgG antibody, PBS (-) solution containing 2% bovine serum albumin (BSA) (product of Seikagaku Corporation) serving as a blocking agent and 0.05% ProClin 300 serving as a preservative was added to each well of the plate, and the plate was left to stand at room temperature for two hours. Thereafter, the plate was washed four times with a washing solution(T-TBS), to thereby obtain a desired a goat anti-mouse IgG antibody immobilized plate.

### (ii) Method of measuring activity of an enzyme-labeled antibody

After completion of washing, T-TBS containing 1% BSA (hereinafter referred to as reaction solution) (100 µL) was added to each well of a goat anti-mouse IgG antibody immobilized plate produced in the above-described (i). Subsequently, to each well, a mouse IgG having a concentration of 100 ng/mL controlled by the reaction solution or the reaction solution serving as a blank was added in an amount of 20 µL, and the mixture was allowed to stand at 37°C for 60 minutes for performing antigen-antibody reaction.

After completion of the reaction, each well was washed four times with T-TBS. Subsequently, each HRP-anti-mouse IgG antibody solution (100 µL) containing a given test substance (stabilizing agent) prepared in the above-described (1) was added to each well, and the mixture was allowed to stand at 37°C for 60 minutes for performing antibody-antigen-antibody reaction.

After completion of the reaction, the plate was washed four times with T-TBS. Subsequently, a tetramethylbenzidine (TMB) solution (100 µL) (product of Moss, inc.) serving as a substrate with respect to peroxidase was added to each well, and the mixture was allowed to react at 37°C for 30 minutes for development. The developed reaction was terminated by adding 1N HCl (100 µL) to each well of the plate, and absorbance of the developed solution formed through degradation of TMB was measured at 450 nm (reference wavelength: 630 nm) by use of a well reader (SK-603, trade name, Seikagaku Corporation).

Reactivity of HRP-anti-mouse IgG was evaluated on the basis of difference in absorbance (i.e., between each sample reacted with mouse IgG (100 ng/mL) and the blank) (hereinafter referred to simply as absorbance difference). With respect to each test substance, stability of HRP-anti-mouse IgG was represented by percent absorbance difference (residual activity; %); i.e., a ratio (×100) of absorbance of a sample at the corresponding test day to absorbance of the sample on the day of preparation of the sample (day 0). When a sample containing a test substance exhibited a residual activity not lower than -1.0 percent point of the residual activity of a control sample containing BSA, the test substance was evaluated to be acceptable, whereas when the sample exhibited a residual activity equal to or higher than the residual activity of the control sample, the test substance was evaluated to have a stabilizing effect equal to or higher than that of BSA. The results are shown in Table 1.

In Example 1, the following commercial products were employed: BSA (Seikagaku Corporation), gelatin and skim milk powder (Nacalai Tesque), and casein (Wako Pure Chemical Industries, Ltd.). Regarding the soybean protein hydrolyzed product, the wheat protein hydrolyzed product, the potato protein hydrolyzed product, and the corn protein hydrolyzed product, the aforementioned soybean protein peptone <2>, soybean protein enzymatically hydrolyzed product <3>, soybean protein acid- hydrolyzed product <4>, wheat protein hydrolyzate <5>, wheat protein hydrolyzate derivative <6>, potato protein hydrolyzate <7>, and corn protein hydrolyzate <8> were employed, respectively

### 2. Results

After storage at 37°C for 12 days, residual activity of HRP-anti-mouse IgG antibody was found to be 4% (no stabilizing agent) and 68% (BSA added). Regarding generally employed animal-derived proteins other than BSA, the residual activity was found to be satisfactory 69% (casein), 14% (gelatin), and 17% (skim milk powder), with the latter two scores being unsatisfactory.

In contrast, when samples contained a plant-derived substance of the present invention (polypeptide), excellent stabilizing effect with a residual activity of 60% or higher was attained with respect to all plant-derived substances tested. Particularly, the corn protein hydrolyzate and the soybean protein hydrolyzate provided a remarkably excellent stabilizing effect; i.e., a residual activity of 86% and 90% or higher, respectively.

The results indicate that a hydrolyzed product of plant-derived protein exhibits an excellent stabilizing effect with respect to the antibody, as compared with animal-derived proteins.

**Table 1**

| | Test substances | | Concentration | Residual activity (%) | | | Rating |
|---|---|---|---|---|---|---|---|
| | | | | day 0 | day 5 | day 12 | |
| Control | | BSA | 1% | 100 | 73 | 68 | BB |
| | | Gelatin | 0.1% | 100 | 40 | 14 | DD |
| | | Casein | 0.1% | 100 | 83 | 69 | BB |
| | | Skim milk powder | 0.1% | 100 | 51 | 17 | DD |
| | | No additives | - | 100 | 24 | 4 | DD |
| Test group | Soybean protein | Peptone | 1% | 100 | 99 | 95 | AA |
| | | Enzymatically hydrolyzed product | 1% | 100 | 101 | 90 | AA |
| | | Acid-hydrolyzed product | 1% | 100 | 88 | 69 | BB |
| | Wheat protein (derivative) | Hydrolyzate | 1% | 100 | 78 | 74 | AA |
| | | Hydrolyzate derivative | 1% | 100 | 80 | 62 | BB |
| | Potato protein | Hydrolyzate | 1% | 100 | 91 | 78 | AA |
| | Corn protein | Enzymatically hydrolyzed product | 1% | 100 | - | 86 | AA |

| | | | | | | | |
|---|---|---|---|---|---|---|---|
| Note. Ratings for residual activity (day 12). DD (0 to 30%), CC (30 to 50%), BB (50 to 70%), and AA (≥70%) | | | | | | | |

### Example 2 (not according to the invention)

Studies on stabilizing effect of the blocking agent of the present invention on an antibody immobilized on a solid phase

### 1. Methods

### <1> Preparation of a blocking agent solution

Each of the test substances (blocking agent) listed in Table 2 was dissolved in a phosphate buffer containing 0.05% ProClin 300 (preservative) (i.e., phosphate buffer; PBS(-) of Example 1 but containing no sodium chloride; hereinafter the buffer referred to as PB(-)) so that a desired test substance concentration was attained. The mixture was filtered by means of a 0.22-µm filter, to thereby obtain a blocking test solution. Each blocking test solution had been stored (2 to 8°C), and the temperature of the solution was adjusted to room temperature before use thereof

### <2> Preparation of a goat anti-mouse IgG antibody immobilized plate and storage thereof

In a manner similar to that of Example 1, a plate was coated with a goat anti-mouse IgG antibody or a normal goat IgG (product of Jackson). A normal goat IgG immobilized plate was employed as a negative control plate. In a similar manner, the plate was washed with PBS(-). In order to block a portion of each well which had not been coated with the antibody, the blocking solution prepared in (1) above serving as a blocking agent was added to each well of the plate, and the plate was left to stand at room temperature for two hours. Thereafter, the solution was removed from the plate, and the plate was dried at 37°C for two hours, to thereby obtain a desired plate immobilized with each antibody.

The thus-prepared plate immobilized with the antibody was placed in an aluminum bag sealable with a zipper. Stability of the plate was investigated on the day of preparation (day 0), 4 days after storage at 55°C (day 4), and 7 days after storage at 55°C (day 7). Each plate immobilized with the antibody was cooled to room temperature before measurement.

### <3> Method of measuring activity of an antibody immobilized on a solid phase

In order to evaluate the stabilizing effect of each blocking solution, the amount (activity) of HRP-labeled mouse IgG which is able to bind to an antibody immobilized on a solid phase was determined in the following manner.

Specifically, the plate immobilized with an antibody prepared in (2) above was cooled to room temperature, and washed four times with T-TBS. Subsequently, a reaction solution (100 µL) containing 200,000-fold diluted HRP-labeled mouse IgG (product of Jackson) was added to each well of the plate, and the mixture was allowed to stand at 37°C for 60 minutes for performing antigen-antibody reaction.

After completion of the reaction, the plate was washed four times with T-TBS. In a manner similar to that of Example 1, a TMB solution (100 µl) (product of Moss, inc.) serving as a substrate with respect to peroxidase was added to each well, and the mixture was allowed to react at 37°C for 30 minutes for development The reaction was terminated by adding 1 mol/L HCl (100 µL) to each well of the plate, and absorbance of the developed solution formed through degradation of TMB was measured at 450 mn (reference wavelength: 630 nm) by use of a well reader (SK-603, trade name, Seikagaku Corporation).

With respect to each blocking solution, reactivity of a goat anti-mouse IgG antibody immobilized on a solid phase (i.e., the amount of HRP-labeled mouse IgG bound to the antibody) was evaluated on the basis of difference in absorbance (i.e., between a sample contained in each well in which a goat anti-mouse IgG antibody had been immobilized on a solid phase and a sample contained in a well in which a normal goat IgG had been immobilized on a solid phase) (hereinafter referred to simply as absorbance difference). With respect to each blocking solution, stability of the goat anti-mouse IgG antibody immobilized on a solid phase was represented by percent absorbance difference (residual activity; %); i.e., a ratio (×100) of absorbance of a sample at the corresponding test day to absorbance of the sample on the day of preparation of the plate immobilized with an antibody (day 0). When the sample exhibited a residual activity of 70% or more, the test blocking solution was evaluated to be excellent. The results are shown in Table 2.

In Example 2, the following commercial products were employed: BSA (Seikagaku Corporation), gelatin and skim milk powder (Nacalai Tesque), and casein (Wako Pure Chemical Industries, Ltd.). Regarding the soybean protein hydrolyzed product, the wheat protein hydrolyzed product, the potato protein hydrolyzed product, the corn protein hydrolyzed product, and the rice bran protein, the aforementioned soybean protein peptone <2>, soybean protein enzymatically hydrolyzed product <3>, soybean protein acid-hydrolyzed product <4>, wheat protein hydrolyzate <5>, wheat protein hydrolyzate derivative <6>, potato protein hydrolyzate <7>, corn protein hydrolyzate <8>, and rice bran protein <9> were employed, respectively.

### 2. Results

After storage at 55°C for 7 days, residual activity of the antibody was found to be 7% (blocked by BSA), which was considerably lowered. Regarding animal-derived proteins generally employed as blocking agents, the residual activity was found to be 27% (gelatin), 40% (casein), and 50% (skim milk powder), which were all higher than the case of blocking by BSA. However, these residual activity values were not satisfactory.

In contrast, when blocking was carried out by a hydrolyzed product of a plant-derived protein, remarkably excellent stabilizing effects on an antibody immobilized on a solid phase with a residual activity of 70% or higher was attained with respect to all hydrolyzed products tested.

The results indicate that a hydrolyzed product of plant-derived protein is useful for a blocking agent which exhibits remarkably excellent stabilizing effects on an antibody immobilized on a solid phase, as compared with animal-derived proteins (or hydrolyzed products thereof).

**Table 2**

| | Test substances | | Concentration | Residual activity (%) | | | Rating |
|---|---|---|---|---|---|---|---|
| | | | | day 0 | day 4 | day 7 | |
| Control | | BSA | 2% | 100 | 12 | 7 | DD |
| | | Gelatin | 0.1% | 100 | 19 | 27 | DD |
| | | Casein | 1% | 100 | 59 | 40 | CC |
| | | Skim milk powder | 1% | 100 | 58 | 50 | CC |
| Test group Test group | Soybean protein | Peptone | 3% | 100 | 95 | 96 | AA |
| | | Enzymatially hydrolyzed product | 3% | 100 | 103 | 95 | AA |
| | | Acid-hydrolyzed product | 1% | 100 | 100 | 97 | AA |
| | Wheat protein (derivative) | Hydrolyzate | 3% | 100 | 100 | 92 | AA |
| | | Hydrolyzate derivative | 3% | 100 | 99 | 96 | AA |
| | Potato protein | Hydrolyzate | 3% | 100 | 96 | 91 | AA |
| | Corn protein | Enzymatially hydrolyzed product | 3% | 100 | 100 | 99 | AA |
| | Rice bran protein | Hydrolyzate | 3% | 100 | 85 | 70 | BB |

| | | | | | | | |
|---|---|---|---|---|---|---|---|
| Note: Ratings for residual activity (day 7): DD (0 to 30%), CC (30 to 50%), BB (50 to 70%), and AA (higher than 70%) | | | | | | | |

### Example 3 (not according to the invention)

Studies on stabilizing effects of the blocking agent of the present invention (i.e., use of blocking agents in combination and use of a blocking agent in combination with another additive) on an antibody

### 1. Methods

### <1> Preparation of blocking solution

In a manner similar to that of Example 2, blocking solutions were prepared by use of the test substances (blocking agents) listed in Table 3.

In Example 3, the same BSA, corn protein (hydrolyzate), rice bran protein (hydrolyzate), and wheat protein (hydrolyzate) as employed in Example 2 were employed (hereinafter these substances are collectively referred to as "base" in the following Tables). The Following blocking agents (stabilizing agents) were also employed: pullulan (product of Seikagaku Corporation); sucrose, maltose, sorbitol lactose, trehalose, raffinose, glycerol, and polyethylene glycol (molecular weight: 300, PEG 300) (products of Wako Pure Chemical Industries, Ltd.); maltooligosaccharide (product name, Fujioligo #360), isomaltooligosaccharide (product name, Panorich), nigerooligosaccharide (product name, Taste Oligo), β-glucooligosaccharide (product name, Gentose), highly branched cyclodextrin (HBCD), and water-soluble cellulose (products of Nihon Shokuhin Kako Co., Ltd.), and carrageenan (product of Sigma).

### <2> Preparation and storage of a goat anti-mouse IgG antibody immobilized plate

In a manner similar to that of Example 1, a goat anti-mouse IgG antibody immobilized plate was prepared by use of each of the blocking solutions prepared in <1> above.

The thus-prepared fabricated plate having the antibody immobilized plate was placed in an aluminum bag sealable with a zipper. Stability of the plate was investigated on the day of preparation (day 0) and 7 days after storage at 55°C (day 7). Each the antibody immobilized plate was cooled to room temperature before measurement.

### <3> Method of measuring activity of an antibody immobilized on a solid phase

In order to evaluate antibody-stabilizing effect of each blocking solution, the amount of the mouse IgG which is able to bind to an antibody immobilized on a solid phase was determined in the following manner.

Specifically, a T-TBS containing 1% BSA (hereinafter referred to as reaction solution) (100 µL) was added to each well of an antibody-immobilized plate prepared in <2> above. The mouse IgG solution (150 ng/mL) prepared by use of the reaction solution was added in an amount of 20 µL to each of the predetermined wells, and an only reaction solution (20 µL) serving as a blank was added to another well. Each mixture was allowed to stand at 4°C for 60 minutes for performing antigen-antibody reaction.

After completion of reaction, the wells were washed four times with a washing solution (T-TBS). An HRP-labeled goat anti-mouse IgG antibody was diluted 5,000-fold with the reaction solution, and the diluted solution (100 µL) was added to each well. The mixture was allowed to stand and react at room temperature (15 to 25°C) for 60 minutes.

After completion of the reaction, the developed reaction procedure employed in Example 1 was repeated, except that the developed reaction was performed at room temperature (15 to 25°C) for 30 minutes. Absorbance of the colored solution formed through degradation of TMB was measured.

Reactivity of the goat anti-mouse IgG antibody immobilized on a solid phase was evaluated on the basis of difference in absorbance (i.e., absorbance of a sample (mouse IgG 150 ng/mL) minus a blank value). Stability of the goat anti-mouse IgG antibody was represented by percent absorbance difference (residual activity; %); i.e., a ratio (×100) of absorbance of a sample on day 7 to absorbance of the sample on the day of preparation of an antibody immobilized plate (day 0). The results are shown in Table 3.

### 2. Results

After storage at 55°C for 7 days (day 7), residual activity of the antibody was found to be 6% (with use of BSA), which is considerably low. When blocking was carried out with corn protein only, a residual activity (serving as an index for a stabilizing effect) as high as 117% was obtained. Use of corn protein in combination with rice bran protein, and use of corn protein in combination with wheat protein provided excellent residual activity scores of 99% and 97%, respectively, indicating that an excellent stabilizing effect was maintained.

In the case where sugar, glycerol, or PEG serving as an additive was used in combination, the obtained residual activity fell within a range of 84 to 119%, indicating that a remarkably excellent stabilizing effect was maintained.

The results indicate that the degraded product of plant-derived protein of the present invention provides long-term, excellent stabilizing effects, when employed singly, in combination of a plurality of proteins, or in combination with another additive.

**Table 3**

| Test substance (blocking agent) | | | | Additive concentration | Residual activity(%) | | Rating |
|---|---|---|---|---|---|---|---|
| Base | Base concentration | Additive | | | day 0 | day 7 | |
| BSA | 2% | None | | - | 100 | 6 | DD |
| Corn protein (hydrolyzate) | 2% | None | | - | 100 | 117 | AA |
| Corn protein (hydrolyzate) | 2% | + | Rice bran protein | 0.1% | 100 | 99 | AA |
| Corn protein (hydrolyzate) | 2% | | Wheat protein | 0.1% | 100 | 97 | AA |
| | | | Sucrose | 1% | 100 | 101 | AA |
| | | | Maltose | 1% | 100 | 108 | AA |
| | | | Sorbitol | 1% | 100 | 107 | AA |
| | | | Lactose | 1% | 100 | 110 | AA |
| | | | Trehalose | 1% | 100 | 106 | AA |
| | | | Raffinose | 1% | 100 | 92 | AA |
| | | | Maltooligosaccharide | 1% | 100 | 104 | AA |
| | | | Isomaltooligasaccharide | 0.1% | 100 | 119 | AA |
| | | | Nigerooligosaccharide | 1% | 100 | 119 | AA |
| | | | β-Gluccoligosaccharide | 1% | 100 | 119 | AA |
| | | | HBCD | 1% | 100 | 92 | AA |
| | | | Water-soluble cellulose | 0.1% | 100 | 84 | AA |
| | | | Pullulan | 1% | 100 | 105 | AA |
| | | | Carrageenan | 0.1% | 100 | 111 | AA |
| | | | Glycerol | 1% | 100 | 97 | AA |
| | | | PEG 300 | 0.1% | 100 | 109 | AA |

| | | | | | | | |
|---|---|---|---|---|---|---|---|
| Note: Ratings for residual activity (day 7): DD (0 to 30%), CC (30 to 50%), BB (50 to 70%), and AA (higher than 70%) | | | | | | | |

### Example 4 (not according to the invention)

Studies on blocking effects of the blocking agent (single use and combination use)

### 1. Methods

### <1> Preparation of blocking solution

In a manner similar to that of Example 2, blocking solutions were prepared by use of test substances (blocking agents) listed in Table 4.

In Example 4, the same corn protein (hydrolyzate) and rice bran protein (hydrolyzate) as employed in Example 2 were employed.

### <2> Preparation and storage of a goat anti-mouse IgG antibody immobilized plate

In a manner similar to that of Example 1, a goat anti-mouse IgG antibody immobilized plate was prepared by use of each of the blocking solutions prepared in <1> above.

### <3> Method of measuring activity of an antibody immobilized on a solid phase

A 50 mmol/L Tris-HCl buffer (Tris-HCl: pH 7.3 to 7.7) containing 0.5% corn protein hydrolyzate, 0.15 mol/L sodium chloride, and 0.05% ProClin 300 (preservative) (hereinafter referred to as TBS reaction solution) (100 µL) was added to each of the predetermined wells of an antibody immobilized plate prepared in <2> above. The mouse IgG solution (6.25 ng/mL) prepared by use of the TBS reaction solution was added in an amount of 25 µL to each well, and a TBS only reaction solution (25 µL) serving as a blank was added to another well. Each mixture was allowed to stand at room temperature (15 to 25°C) for 60 minutes for performing antigen-antibody reaction.

After completion of reaction, the wells were washed four times with a washing solution (T-TBS). An HRP-labeled goat anti-mouse IgG antibody was diluted 1,000-fold with the T-TBS reaction solution. The TBS reaction solution or the above diluted HRP-labeled antibody (100 µL) was added to each well. The mixture was allowed to stand and react at room temperature (15 to 25°C) for 60 minutes.

After completion of the reaction, absorbance of each sample was measured in a manner similar to that of Example 3.

The amount of IgG which had been non-specifically adsorbed was represented by the absorbance of the blank. The amount IgG which had been specifically adsorbed to the antibody was evaluated on the basis of difference in absorbance (hereinafter referred to as absorption difference) (i.e., absorbance of a sample (mouse IgG 6.25 ng/mL) minus a blank value).

### 2. Results

When no blocking agent was used, the blank exhibited an absorbance as high as 2.963, indicating that non-specific adsorption occurred considerably. In contrast, when corn protein was used singly, an absorbance of 0.149 was obtained, whereas when corn protein was used in combination with rice bran protein serving as an additive (concentration: 0.01 to 2%), an absorbance of 0.059 to 0.118 was obtained. When any of the blocking agents was used, the blank value was maintained at low level. The results indicate that the blocking agent, either in the case of single use or combination use, remarkably suppresses non-specific adsorption of IgG.

The absorbance difference, which is an index for evaluating specific adsorption, was found to be 0.025 when no blocking agent was employed. The low value was attributed to failure to effectively detect specific adsorption. In contrast, when corn protein was used singly, an absorbance difference of 0.585 was obtained, whereas when corn protein was used in combination with rice bran protein serving as an additive (concentration: 0.01 to 2%), an absorbance difference of 0.680 to 0.929 was obtained. The results indicate that the blocking agent, either in the case of single use or combination use, allows detection of specific adsorption at high efficiency

As describe hereinabove, the blocking agent has been confirmed to prevent non-specific adsorption of an antigen onto an antibody-immobilized plate and to enhance reactivity of antigen-antibody reaction.

**Table 4**

| Test substance (blocking agent) | | | | Absorbance (A) | | ΔA |
|---|---|---|---|---|---|---|
| Base | Base concentration | Additive | Additive concentration | IgG | Blank | |
| None | - | None | - | 2.988 | 2.963 | 0.025 |
| Corn protein (hydrolyzate) | 2% | None | - | 0.734 | 0.149 | 0.585 |
| Corn protein (hydrolyzate) | 2% | + Rice bran protein (hydrolyzate) | 0.01% | 0.831 | 0.118 | 0.713 |
| | | | 0.05% | 0.928 | 0.059 | 0.869 |
| | | | 0.1% | 0.830 | 0.065 | 0.765 |
| | | | 0.2% | 0.865 | 0.078 | 0.787 |
| | | | 0.5% | 0.771 | 0.091 | 0.680 |
| | | | 1% | 0.915 | 0.103 | 0.812 |
| | | | 2% | 1.033 | 0.104 | 0.929 |

| | | | | | | |
|---|---|---|---|---|---|---|
| Note: ΔA; absorbance difference | | | | | | |

As described hereinabove, the present invention provides the use of a stabilizing agent for a specific-binding-pair-forming substance; and the use of a composition in an assay employing a specific-binding-pair-forming substance, wherein the composition comprises the specific-binding-pair-forming substance and the stabilizing agent. The stabilizing agent used in the present invention exerts excellent stabilizing effects for a specific-binding-pair-forming substance as compared with BSA, which is a known stabilizing agent, and which does not raise any problem of contamination with pathogens.

## Claims

1. Use of a plant-derived polypeptide product obtained from the degradation of a plant-derived protein which is selected from a soybean protein hydrolyzate, a wheat protein hydrolyzate and derivatives thereof, a potato protein hydrolyzate, a corn protein hydrolyzate, and a rice bran protein hydrolyzate as a stabilizing agent in a method for stabilizing a specific-binding-pair-forming substance selected from an antibody or an antigen stored in a solution,
wherein said polypeptide product is in an aqueous solution form containing an alkali or buffer.

2. The use according to claim 1, wherein the method for stabilizing said specific-binding-pair-forming substance comprises bringing said polypeptide product as a stabilizing agent into coexistence with said specfic-binding-pair-forming substance.

3. The use according to claim 2, wherein the antibody or the antigen are in a complex with a labeling substance, which is selected from an enzyme, avidin, streptoavidin, biotin, a radioisotope, a fluorescent substance, a luminescence substance, and metal colloid, for use in an Immunoassay.

4. The use according to claim 1, wherein the solution has a pH of 3.0 to 9.0.

5. The use according to claim 1 or 4, wherein the solution is sterilized through filter-sterilization.

## Patentansprüche

1. Verwendung eines von Pflanzen abgeleiteten Polypeptid-Produkts, erhalten aus dem Abbau eines von Pflanzen abgeleiteten Proteins, das aus einem Sojabohnenprotein-Hydrolysat, einem Weizenprotein-Hydrolysat und dessen Derivaten, einem Kartoffelprotein-Hydrolysat, einem Maisprotein-Hydrolysat und einem Reiskleieprotein-Hydrolysat ausgewählt ist, als stabilisierendes Mittel in einem Verfahren zur Stabilisierung einer ein spezifisches bindendes Paar bildenden Substanz, die aus einem in einer Lösung aufbewahrten Antikörper oder Antigen ausgewählt ist, wobei das Polypeptid-Produkt in einer wässrigen Lösungsform vorliegt, die ein Alkali oder einen Puffer enthält.

2. Verwendung nach Anspruch 1, bei der das Verfahren zur Stabilisierung der ein spezifisches bindendes Paar bildenden Substanz umfasst, dass man das Polypeptid-Produkt als stabilisierendes Mittel in die Gegenwart einer ein spezifisches bindendes Paar bildenden Substanz bringt.

3. Verwendung nach Anspruch 2, bei der der Antikörper oder das Antigen in einem Komplex mit einer Markierungssubstanz, die aus einem Enzym, Avidin, Streptoavidin, Biotin, einem Radioisotop, einer fluoreszierenden Substanz, einer lumineszierenden Substanz und Metallkolloid ausgewählt ist, zur Verwendung in einem Immunoassay vorliegt.

4. Verwendung nach Anspruch 1, bei der die Lösung einen pH von 3,0 bis 9,0 aufweist.

5. Verwendung nach Anspruch 1 oder 4, bei der die Lösung durch Filtersterilisation sterilisiert ist.

## Revendications

1. Utilisation d'un produit polypeptidique d'origine végétale obtenu par la dégradation d'une protéine d'origine végétale qui est sélectionnée parmi un hydrolysat de protéine de soja, un hydrolysat de protéine de blé et des dérivés de ceux-ci, un hydrolysat de protéine de pomme de terre, un hydrolysat de protéine de maïs et un hydrolysat de protéine de son de riz en tant qu'agent stabilisant dans un procédé de stabilisation d'une substance formant des paires liantes spécifiques sélectionnée parmi un anticorps ou un antigène stocké dans une solution,
ledit produit polypeptidique étant sous la forme d'une solution aqueuse contenant un alcali ou un tampon.

2. Utilisation selon la revendication 1, dans laquelle le procédé de stabilisation de ladite substance formant des paires liantes spécifiques consiste à faire coexister ledit produit polypeptidique en tant qu'agent stabilisant avec ladite substance formant des paires liantes spécifiques.

3. Utilisation selon la revendication 2, dans laquelle l'anticorps ou l'antigène est en complexe avec une substance marquante qui est sélectionnée parmi une enzyme, de l'avidine, de la streptavidine, de la biotine, un radio-isotope, une substance fluorescente, une substance luminescente et un colloïde métallique utilisable dans un immunoessai.

4. Utilisation selon la revendication 1, dans laquelle la solution a un pH compris entre 3,0 et 9,0.

5. Utilisation selon la revendication 1 ou 4, dans laquelle la solution est stérilisée par stérilisation par filtration.
